(19)

Europäisches
Patentamt
European
Patent Office
Office européen
des brevets

(11) **EP 3 100 700 A1**

(12) **EUROPEAN PATENT APPLICATION**

(43) Date of publication:
07.12.2016 Bulletin 2016/49

(21) Application number: 16158797.7

(22) Date of filing: 04.03.2016

(51) Int Cl.:
*A61F 2/07* (2013.01)     *A61F 2/06* (2006.01)
*A61B 5/00* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 01.06.2015 JP 2015111501

(71) Applicant: Kawasumi Laboratories, Inc.
Oita 876-0121 (JP)

(72) Inventors:
• NOMIYAMA, Hiroaki
  Minato-ku, Tokyo 108-6109 (JP)
• AKITA, Kazumi
  Usuki-shi, Oita 875-0203 (JP)

(74) Representative: Reeve, Nicholas Edward
Reddie & Grose LLP
16 Theobalds Road
London WC1X 8PL (GB)

(54) **TUBULAR ENDOPROSTHESIS DESIGNING APPARATUS, TUBULAR ENDOPROSTHESIS MANUFACTURING METHOD, AND TUBULAR ENDOPROSTHESIS DESIGNING PROGRAM**

(57)     A tubular endoprosthesis designing apparatus that designs a stent graft to be housed in a sheath comprises a three-dimensional model generating unit, a simulation unit, and an opening position determination unit. The three-dimensional model generating unit generates a three-dimensional model of a blood vessel in which a stent graft is to be implanted. The simulation unit simulates a state in which a sheath is inserted into the three-dimensional model generated by the three-dimensional model generating unit. The opening position determination unit determines the position of the opening to be formed in the tubular wall of the stent graft based on the simulation result obtained by the simulation unit and the information with respect to the shape of twisting of the blood vessel in a region in which the stent graft is to be implanted.

```
                                        100
 ┌─────────────────────────────────────────────┐
 │ TUBULAR ENDOPROSTHESIS MANUFACTURING SYSTEM  │
 │  ┌─────────────────────────────────────┐     │
 │  │ TUBULAR ENDOPROSTHESIS DESIGNING     │ ~1  │
 │  │           APPARATUS                  │     │
 │  │  ┌────────────────────────────────┐  │     │
 │  │  │ THREE-DIMENSIONAL MODEL        │~10│     │
 │  │  │ GENERATING UNIT                │  │     │
 │  │  └────────────────────────────────┘  │     │
 │  │  ┌────────────────────────────────┐  │     │
 │  │  │ SIMULATION UNIT                │~20│     │
 │  │  └────────────────────────────────┘  │     │
 │  │  ┌────────────────────────────────┐  │     │
 │  │  │ OPENING POSITION DETERMINATION │~30│     │
 │  │  │ UNIT                           │  │     │
 │  │  └────────────────────────────────┘  │     │
 │  └─────────────────────────────────────┘     │
 │  ┌─────────────────────────────────────┐     │
 │  │ TUBULAR ENDOPROSTHESIS MANUFACTURING │ ~2  │
 │  │           APPARATUS                  │     │
 │  └─────────────────────────────────────┘     │
 └─────────────────────────────────────────────┘
```

FIG. 1

EP 3 100 700 A1

**Description**

Technical Field

The present invention relates to a tubular endoprosthesis designing apparatus, a tubular endoprosthesis manufacturing method, and a tubular endoprosthesis designing program.

Background of the Invention

As a stent graft employed in stent graft implantation according to conventional techniques, a stent graft is known configured to have an opening (fenestration, side hole) in its tubular wall (see Japanese Patent Application Laid Open No. 2010-227486 and Japanese Patent Application Laid Open No. 2013-52282, for example). In a case in which there is an aneurysm in the vicinity of an orifice of a branched vessel, by placing a stent graft having such an opening at such a lesion, such an arrangement is capable of blocking the flow of blood to the aneurysm without inhibiting the flow of blood to the branched vessel.

In the process of manufacturing such a stent graft having an opening, before the stent graft implantation, first, two-dimensional CT (Computed Tomography) images are acquired for the blood vessels of the patient, for example. Subsequently, three-dimensional blood vessel images are constructed by means of software based on the two-dimensional blood vessel images thus acquired. Next, a stent graft having an optimal structure is designed according to the blood vessel structure in a region in which the stent graft is to be implanted, based on the two-dimensional blood vessel images and the three-dimensional blood vessel images. Furthermore, the stent graft is designed to have an opening at the optimal position such that the stent graft does not inhibit the blood that flows through the branched vessel after the stent graft is implanted at a lesion.

However, it is needless to say that there are individual differences in blood vessel structure. That is to say, there is a difference in the position and the shape of an aneurysm among patients. Accordingly, it is difficult to design the optimal position of such an opening without a doctor's many years of experience or otherwise technical support based on experience.

Such a problem can also occur in other kinds of tubular endoprostheses in addition to such a stent graft, examples of which include a synthetic graft having no stent framework.

Accordingly, we have appreciated that it would be desirable to address the aforementioned problem, and provide a technique for allowing the designer to easily design a tubular endoprosthesis having an opening at an optimal position in its tubular wall.

SUMMARY OF THE INVENTION

As a result of investigations, the present inventors have found that there is a difference in the position of a sheath after it is inserted into a blood vessel, according to the shape of curvature of the blood vessel in a region in which the tubular endoprosthesis is to be implanted. This has a large effect on the implantation position (position deviation) of the tubular endoprosthesis. In order to solve such a problem, it has been found that, by simulating the insertion of the sheath into the blood vessel after the information with respect to the shape of curvature of the blood vessel is acquired, and by determining the position of the opening based on the simulation results, such an arrangement is capable of determining an optimal position of the opening formed in the tubular wall of the tubular endoprosthesis in a simple manner, thereby achieving the present invention.

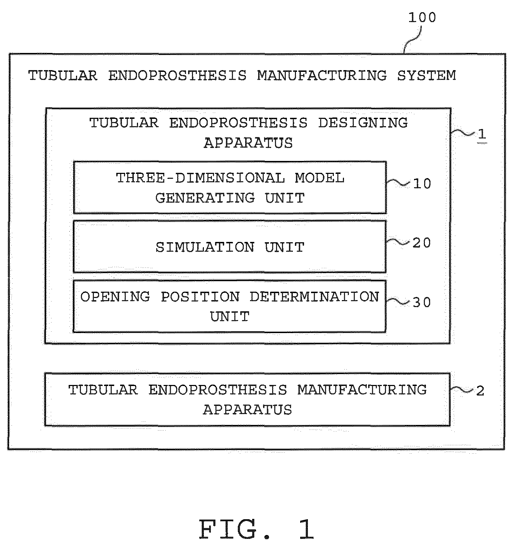

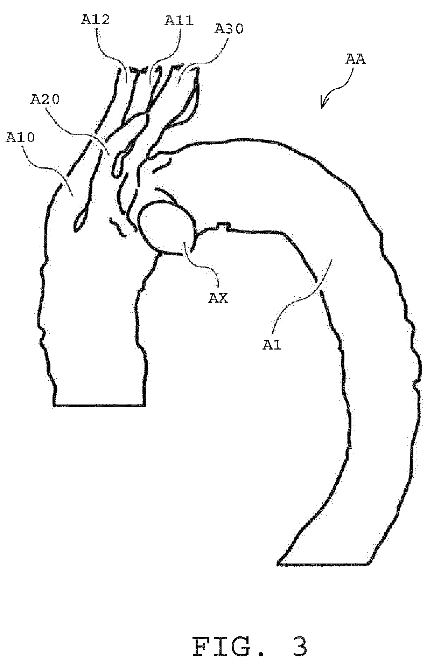

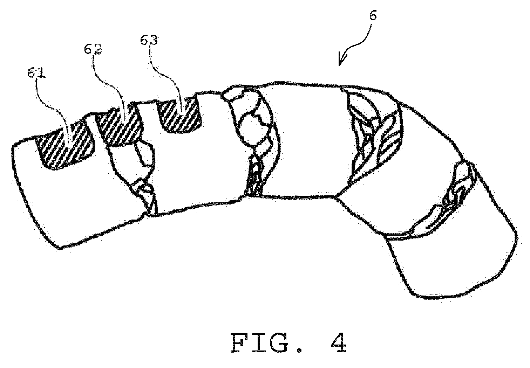

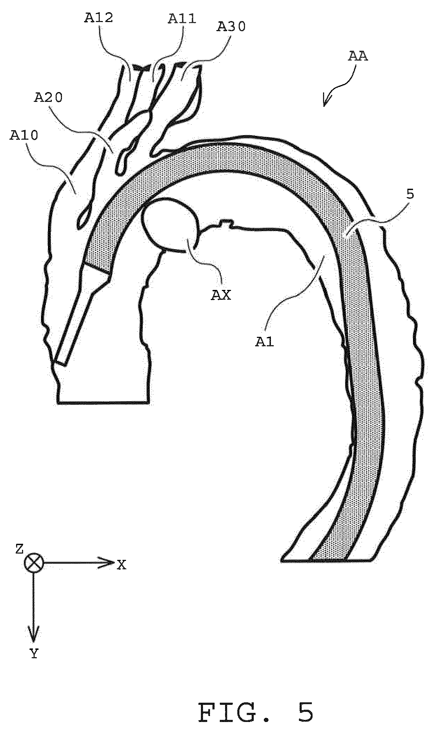

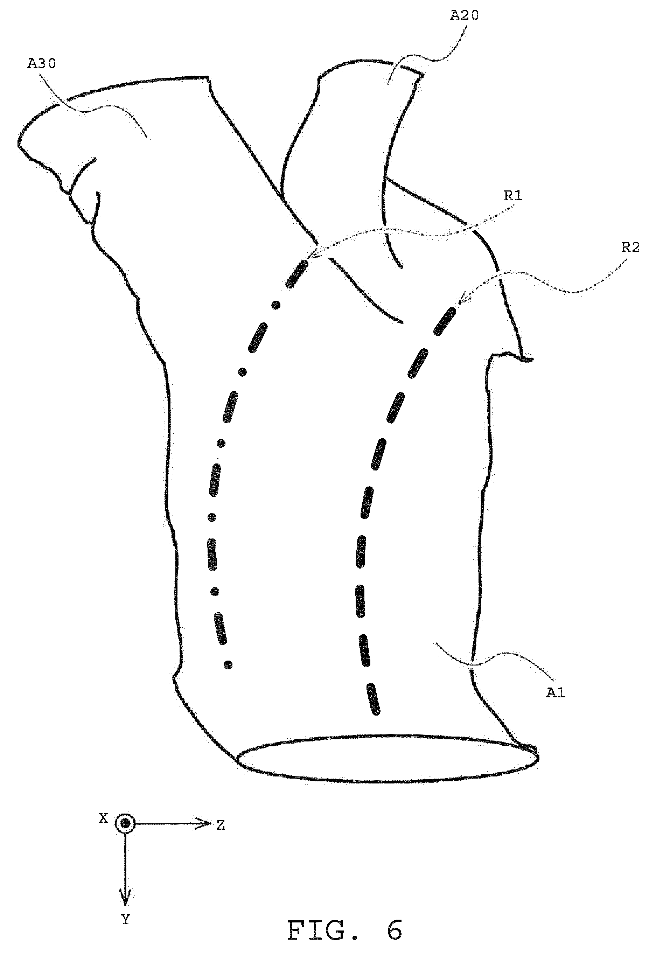

(1) The present invention proposes a tubular endoprosthesis designing apparatus (which corresponds to the tubular endoprosthesis designing apparatus 1 shown in Fig. 1, for example) that designs a tubular endoprosthesis (which corresponds to the stent graft 6 shown in Fig. 4, for example) to be implanted in a body after it is housed in a sheath (which corresponds to the sheath 5 shown in Fig. 5, for example). The tubular endoprosthesis is to be implanted in a region including an orifice of a branched vessel (which corresponds to the orifice of the brachiocephalic artery A10, the left common carotid artery A20, or the left subclavian artery A30, shown in Fig. 3, for example). The tubular endoprosthesis designing apparatus comprises: a three-dimensional model generating unit (which corresponds to the three-dimensional model generating unit 10 shown in Fig. 1, for example) that generates a three-dimensional model (which corresponds to the three-dimensional model AA shown in Fig. 3, for example) of a blood vessel in which the tubular endoprosthesis is to be implanted; a simulation unit (which corresponds to the simulation unit 20 shown in Fig. 1, for example) that simulates a state in which the sheath is inserted into the three-dimensional model generated by the three-dimensional model generating unit; and an opening position determination unit (which corresponds to the opening position determination unit 30 shown in Fig. 1, for example) that determines a position of an opening (which corresponds to the opening 61, 62, or 63, shown in Fig. 4, for example) to be formed in a tubular wall of the tubular endoprosthesis, based on a simulation result obtained by the simulation unit and information with respect to a shape of curvature of the blood vessel (which corresponds to the shape of twisting of the blood vessel described later, for example) in which the tubular endoprosthesis is to be implanted. The sheath is configured such that it is curved in a predetermined single direction or otherwise can be curved. The information used by the opening position determination unit is a position relation between an edge line of the blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result by the simulation unit.

With such an arrangement according to the invention, the three-dimensional model generating unit generates a three-dimensional model of a blood vessel in which a tubular endoprosthesis is to be implanted. Furthermore, the simulation unit simulates a state in which a sheath is inserted into the three-dimensional model thus generated by the three-dimensional model generating unit. Moreover, the opening position determination unit determines the position of each opening to be formed in the tubular wall of the tubular endoprosthesis, based on the simulation result obtained by the simulation unit and the information with respect to the shape of curvature of the blood vessel in a region in which the endoprosthesis is to be implanted. Thus, even if there is an effect on the implantation position (deviation) of the tubular endoprosthesis due to individual differences in the shape of curvature of the blood vessel among patients, such an arrangement is capable of determining the position for each opening to be formed giving consideration to this effect due to such individual differences. Such an arrangement is capable of determining, in a simple manner, the optimal position for each opening to be formed in the tubular endoprosthesis.

Furthermore, with such an arrangement according to the present invention, the position of each opening to be formed is determined based on the position relation between the edge line of the blood vessel in a region in which the tubular endoprosthesis is to be implanted and the edge line of the sheath in a state in which it is inserted into this region, detailed description of which will be made later. Thus, such an arrangement is capable of determining an optimal position of the opening to be formed, without being concerned about the degree of curvature of the blood vessel.

(2) The present invention proposes the tubular endoprosthesis designing apparatus described in (1). With such an arrangement, the opening position determination unit defines, as a twisting of a blood vessel, a deviation of the blood vessel in a direction that is orthogonal to a direction of curvature of the blood vessel is curved (which corresponds to a direction along the Z axis shown in Fig. 5, for example). Also, the opening position determination unit uses, as a shape of curving of the blood vessel in the region in which the tubular endoprosthesis is to be implanted, a shape of twisting of the blood vessel in the region in which the tubular endoprosthesis is to be implanted.

Thus, with such an arrangement according to the present invention, such an arrangement is capable of determining an optimal position of the opening to be formed in the tubular wall of the tubular endoprosthesis giving consideration to the shape of twisting of the blood vessel.

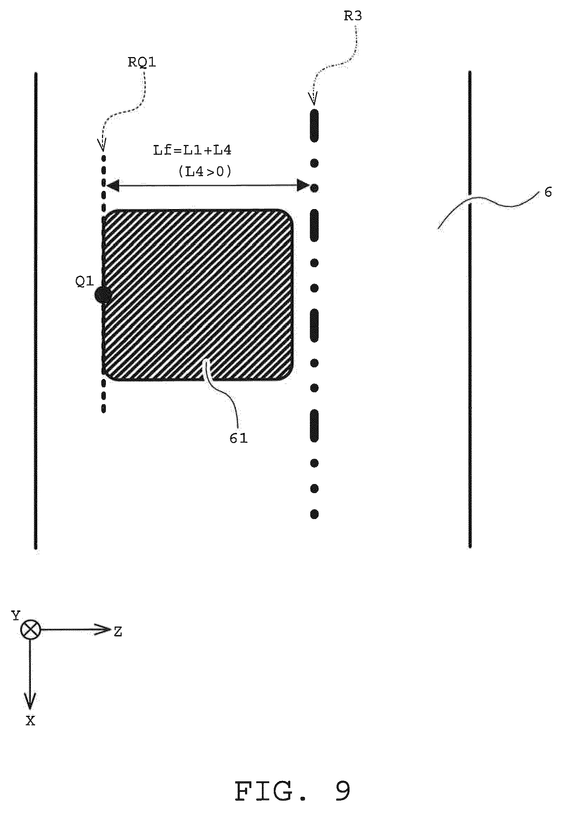

(3) The present invention proposes the tubular endoprosthesis designing apparatus described in (1) or (2). The tubular endoprosthesis is configured such that it is curved in a predetermined direction or otherwise such that it can be curved. Furthermore, the tubular endoprosthesis is housed in the sheath in a state in which the direction in which the tubular endoprosthesis is curved matches the direction in which the sheath is curved. The information used by the opening position determination unit comprises: a position relation between an edge line of a blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result by the simulation unit; and a position relation between an edge line of the sheath (which corresponds to the edge line R2 of the sheath 5 shown in Fig. 6, for example) in a state in which it is inserted into a region in which the tubular endoprosthesis is to be implanted and an edge line of the tubular endoprosthesis (edge line R3 of the stent graft 6 shown in Fig. 9, for example) in a state in which it is implanted in the aforementioned region after it is deployed from the sheath, which is obtained as a simulation result by the simulation unit.

With such an arrangement according to the present invention, the position of each opening to be formed is determined based on the position relation between the edge line of the blood vessel in a region in which the tubular endoprosthesis is to be implanted and the edge line of the sheath in a state in which it is inserted into this region, and the position relation between the edge line of the sheath and the edge line of the tubular endoprosthesis in a state in which it is implanted in this region after it is deployed from the sheath. Thus, such an arrangement is capable of determining, with higher precision, an optimal position of the opening to be formed, without being concerned about the degree of curvature of the blood vessel.

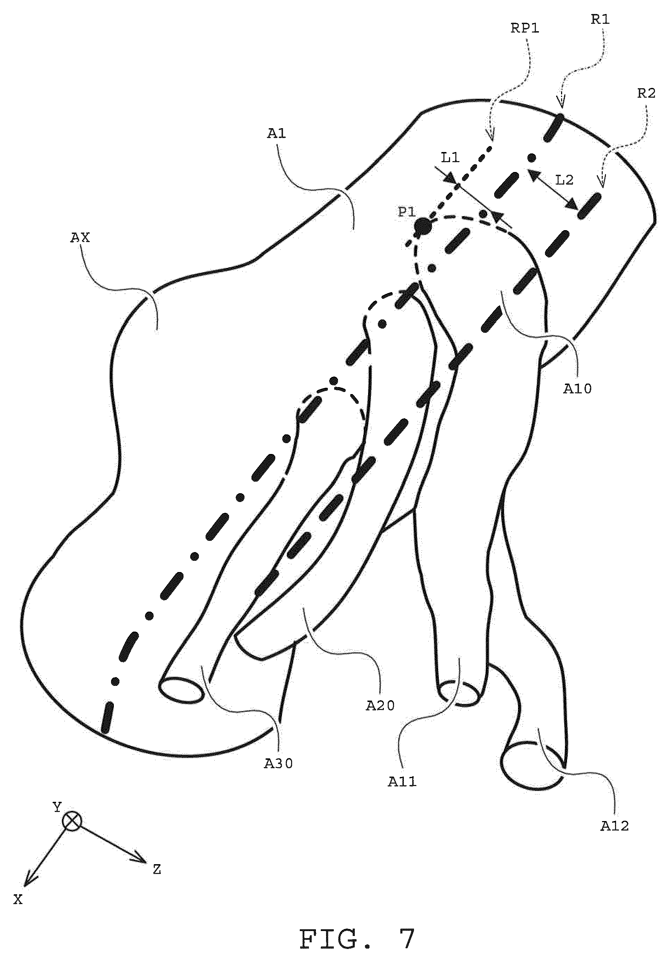

(4) The present invention proposes the tubular endoprosthesis designing apparatus described in (3). With such an arrangement, the opening position determination unit defines, as L1, a shortest distance between an edge line of a blood vessel (which corresponds to the edge line R1 of the thoracic aorta A1 shown in Fig. 6, for example) in a region in which the tubular endoprosthesis is to be implanted and a reference position defined on an orifice of a branched vessel (which corresponds to edge point P1 of the orifice of the brachiocephalic artery A10 shown in Fig. 7, for example), which is obtained as a simulation result by the simulation unit. Furthermore, the opening position determination unit defines, as L2, a position deviation between an edge line of the blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result by the simulation unit. Moreover, the opening position determination unit defines, as L3, a position deviation between an edge line of the sheath (which corresponds to the edge line R2 of the sheath 5 shown in Fig. 6, for example) in a state in which it is inserted into a region in which the tubular endoprosthesis is to be implanted and an edge line of the tubular endoprosthesis (edge line R3 of the stent graft 6 shown in Fig. 9, for example) in a state in which it is implanted in the aforementioned region after it is deployed from the sheath, which is obtained as a simulation result by the simulation unit. In this state, the opening position determination unit determines a position of the opening such that a shortest distance Lf between an edge line of the tubular endoprosthesis in a state in which it is implanted in the aforementioned region and a reference position defined on the opening satisfy the following expression (Expression 1).

Expression 1

$$Lf = L1 + L2 + L3 \quad \cdots \quad (1)$$

With such an arrangement according to the present invention, such an arrangement is capable of determining, in a relatively simple manner, an optimal position of each opening to be formed, using the aforementioned Expression (1).

(5) The present invention proposes the tubular endoprosthesis designing apparatus described in (1) or (2). With such an arrangement, the opening position determination unit defines, as L1, a shortest distance between an edge line of a blood vessel (which corresponds to the edge line R1 of the thoracic aorta A1 shown in Fig. 6, for example) in a region in which the tubular endoprosthesis is to be implanted and a reference position defined on an orifice of a branched vessel (which corresponds to edge point P1 of the orifice of the brachiocephalic artery A10 shown in Fig. 7, for example), which is obtained as a simulation result by the simulation unit. Furthermore, the opening position determination unit defines, as L2, a position deviation between an edge line of the blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result by the simulation unit. In this state, the opening position determination unit determines a correction value L4 based on the position deviation L2. Moreover, the opening position determination unit determines a position of the opening such that a shortest distance Lf between an edge line of the tubular endoprosthesis in a state in which it is implanted in the aforementioned region and a reference position defined on the opening satisfy the following expression (Expression 2).

Expression 2

$$Lf = L1 + L4 \quad \cdots \quad (2)$$

With such an arrangement according to the present invention, the relation between the position deviation L2 and the correction value L4 is determined beforehand. Thus, such an arrangement is capable of determining, in a simpler manner, an optimal position of each opening to be formed, using the aforementioned Expression (2).

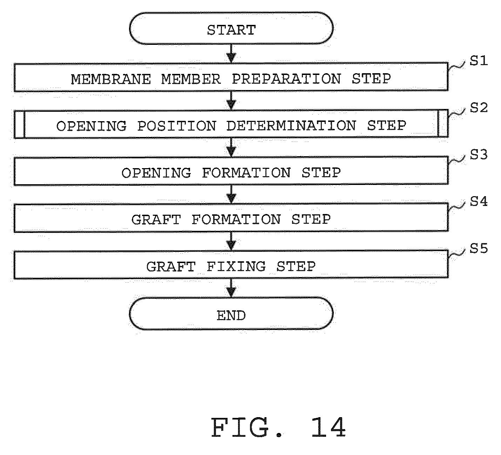

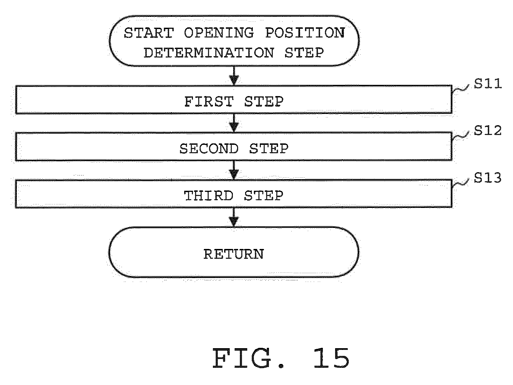

(6) The present invention proposes a manufacturing method (which corresponds to the manufacturing method shown in Fig. 14, for example) for a tubular endoprosthesis (which corresponds to the stent graft 6 shown in Fig. 4, for example) to be implanted in a body after being housed in a sheath (which corresponds to the sheath 5 shown in Fig. 5, for example). The sheath is configured such that it is curved in a predetermined single direction or otherwise such that it can be curved. The tubular endoprosthesis is to be implanted in a region including an orifice of a branched vessel (which corresponds to the orifice of the brachiocephalic artery A10, the left common carotid artery A20, or the left subclavian artery A30, shown in Fig. 3, for example). The manufacturing method comprises: a membrane member preparation step (which corresponds to Step S1 shown in Fig. 14, for example) in which a membrane member to be configured as the tubular endoprosthesis is prepared; an opening position determination step (which corresponds to Step S2 shown in Fig. 14, for example) in which a position is determined for an opening (which corresponds to the opening 61, 62, or 63, shown in Fig. 4, for example) to be formed in a tubular wall of the membrane member; an opening formation step (which corresponds to Step S3 shown in Fig. 14, for example) in which the opening is formed in the membrane member prepared in the membrane member preparation step such that it is formed at a position determined in the opening position determination step. The opening position determination step comprises: a first step (which corresponds to Step S11 shown in Fig. 15, for example) in which a three-dimensional model (which corresponds to the three-dimensional model AA shown in Fig. 3, for example) is generated for a blood vessel in which the tubular endoprosthesis is to be implanted; a second step (which corresponds to Step S12 shown in Fig. 15, for example) in which a state is simulated in which the sheath is inserted into the three-dimensional model generated in the first step; and a third step (which corresponds to Step S13 shown in Fig. 15, for example) in which the position of the opening is determined based on a simulation result obtained in the second step and information with respect to a shape of curvature of the blood vessel (which corresponds to the shape of twisting of the blood vessel described later) in which the tubular endoprosthesis is to be implanted. The information used in the third step is a position relation between an edge line of the blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result in the second step. With such an arrangement according to the present invention, such an arrangement provides the same effects as those provided by the tubular endoprosthesis designing apparatus described in (1) according to the invention. Furthermore, such an arrangement is capable of manufacturing a tubular endoprosthesis having openings each of which is formed at its optimal position. It should be noted that the manufacturing method for a tubular endoprosthesis described in (6) may further comprise the same technical features as those provided to the tubular endoprosthesis designing apparatus described in any one of (2) through (5).

(7) The present invention proposes a tubular endoprosthesis designing program configured to instruct a computer to execute a tubular endoprosthesis designing method for designing a tubular endoprosthesis (which corresponds to the stent graft 6 shown in Fig. 4, for example) that is to be implanted in a body and that is to be housed in a sheath (which corresponds to the sheath 5 shown in Fig. 5, for example). The sheath is configured such that it is curved in a predetermined single direction or otherwise such that it can be curved. The tubular endoprosthesis is to be implanted in a region including an orifice of a branched vessel (which corresponds to the orifice of the brachiocephalic artery A10, the left common carotid artery A20, or the left subclavian artery A30, shown in Fig. 3, for example). The tubular endoprosthesis designing program executed by the computer comprises: a first step (which corresponds to Step S11 shown in Fig. 15, for example) in which a three-dimensional model (which corresponds to the three-dimensional model AA shown in Fig. 3, for example) is generated for a blood vessel in which the tubular endoprosthesis is to be implanted; a second step (which corresponds to Step S12 shown in Fig. 15, for example) in which a state is simulated in which the sheath is inserted into the three-dimensional model generated in the first step; and a third step (which corresponds to Step S13 shown in Fig. 15, for example) in which the position of the opening to be formed in a tubular wall of the tubular endoprosthesis is determined based on a simulation result obtained in the second step and information with respect to a shape of curvature of the blood vessel (which corresponds to a shape of twisting of the blood vessel described later) in which the tubular endoprosthesis is to be implanted. The information used in the third step is a position relation between an edge line of the blood vessel (which corresponds to the edge line R1 of the thoracic aorta A1 shown in Fig. 6, for example) in a region in which the tubular endoprosthesis is to be implanted and an edge line of the sheath (which corresponds to the edge line R2 of the sheath 5 shown in Fig. 6, for example) in a state in which it is inserted into the aforementioned region, which is obtained as a simulation result in the second step.

With such an arrangement according to the present invention, a computer is instructed to execute the tubular endoprosthesis designing program described in (7), thereby providing the same effects as those provided by the tubular endoprosthesis designing apparatus described in (1) according to the invention. It should be noted that the tubular endoprosthesis designing program described in (7) may further comprise the same technical features as those provided to the tubular endoprosthesis designing apparatus described in any one of (2) through (5).

BRIEF DESCRIPTION OF THE DRAWINGS

It should be noted that description will be made regarding an embodiment of the present invention with reference to the corresponding symbols. However, the present invention is not restricted to such an embodiment.

Fig. 1 is a block diagram showing a tubular endoprosthesis manufacturing system according to a first embodiment of the present invention.

Fig. 2 is a block diagram showing a computer that provides a tubular endoprosthesis designing apparatus according to the first embodiment of the present invention.

Fig. 3 is a three-dimensional model generated by a three-dimensional model generating unit according to the first embodiment of the present invention.

Fig. 4 is an external perspective view of a stent graft.

Fig. 5 is a diagram showing a state in which a sheath is inserted into a three-dimensional model.

Fig. 6 is a diagram showing the position relation between an edge line of a thoracic aorta and an edge line of a sheath, which is acquired by a simulation unit as a simulation result.

Fig. 7 is a diagram showing the position relation between an edge line of a thoracic aorta and an edge line of a sheath, which is acquired by a simulation unit as an simulation result.

Fig. 8 is a diagram showing the position relation between an edge line of a thoracic aorta and an edge line of a sheath.

Fig. 9 is a diagram showing the position relation between the edge line of a stent graft and an opening.

Fig. 10 is a diagram showing the position relation between an edge line of a thoracic aorta and an edge line of a sheath.

Fig. 11 is a diagram showing the position relation between an edge line of a stent graft and an opening.

Fig. 12 is a diagram showing the position relation between an edge line of a thoracic aorta and an edge line of a sheath.

Fig. 13 is a diagram showing the position relation between an edge line of a stent graft and each opening.

Fig. 14 is a flowchart showing a manufacturing method for a stent graft according to the first embodiment of the present invention.

Fig. 15 is a flowchart showing an opening position determination step according to the first embodiment of the present invention.

Fig. 16 is a block diagram showing a tubular endoprosthesis manufacturing system according to a second embodiment of the present invention.

## DETAILED DESCRIPTION OF THE EXAMPLE EMBODIMENTS

First, description will be made below regarding the outline of the present invention.

The present inventors constructs a blood vessel model based on the blood vessel images, and designed and formed a stent graft based on the same blood vessel images. Furthermore, the present inventors conducted an experiment of implanting the stent graft thus formed in the blood vessel model thus formed. As a result of the experiment, the present inventors have found that, as the degree of twisting of the blood vessel in the vicinity of the branched vessel becomes larger, it becomes more difficult to provide alignment between an opening (fenestration, side hole) of the stent graft and the orifice of the branched vessel.

Accordingly, the present inventors have accumulated research on the cause of the mechanism in which, as the degree of twisting of a blood vessel in the vicinity of a branched vessel becomes larger, it becomes more difficult to provide alignment between the opening of the stent graft and the orifice of the branched vessel. As a result of these investigations, the present inventors have found that, in a case of employing a sheath formed in a shape that is curved in a predetermined single direction, or otherwise a sheath formed such that it can be curved in a predetermined single direction, as a sheath for housing a stent graft, there is a difference in the trajectory of the sheath when it passes through a blood vessel according to the degree of twisting of the blood vessel. This has a large effect on the implantation position (position deviation) of the stent graft.

Description will be made below regarding the "twisting of the blood vessel" described above. In a case in which the "blood vessel" is the thoracic aorta, in some cases, the blood vessel is curved not only in a U shape (inverted U shape) on a virtual plane, but also curved in a front-back direction (forward direction or otherwise backward direction), i.e., in a direction that is orthogonal to the direction in which the blood vessel is curved on the virtual plane. The aforementioned "twisting of the blood vessel" represents the shape of curvature of the blood vessel in the front-back direction. That is to say, in some cases, in addition to a curve in a virtual plane, a given blood vessel has another curve in a direction having a vector component that is orthogonal to the virtual plane. That is to say, the "twisting of the blood vessel" represents a curve of the blood vessel in a direction having a vector component that is orthogonal to the aforementioned virtual plane.

In a case in which a blood vessel has relatively slight twisting (in a case in which there is almost no twisting in the blood vessel), the sheath is passed through the interior of the blood vessel along its center axis. Accordingly, a stent graft deployed from the sheath is implanted within the blood vessel such that the edge of the stent graft with greater curvature approximately matches the edge of the blood vessel with greater curvature.

In contrast, an experiment conducted by the present inventors shows that, in a case in which there is relatively severe twisting in a blood vessel, the sheath does not always pass through the blood vessel along its center axis. That is to say, it has been found that, in some cases, the sheath deviates from the center axis of the blood vessel when it passes through a region in which the blood vessel has severe twisting. In a case of employing a stent graft having curvature in a predetermined single direction, and in a case in which such a stent graft is housed in the sheath such that the direction of curvature of the stent graft matches the direction of curvature of the sheath, in some cases, the stent graft is deployed from the sheath in a state in which it has deviated from the center axis of the blood vessel. In this case, there is a low probability that the stent graft will be implanted within the blood vessel such that the edge of the stent graft with greater curvature matches the edge of the blood vessel with greater curvature.

In a case in which a blood vessel has relatively slight twisting, before an opening is formed in the tubular wall of the stent graft, for example, the distance between the edge of the blood vessel with greater curvature and the orifice of the branched vessel (which corresponds to the "shortest distance L1" described later) is calculated, and the position of the opening is determined such that the distance thus calculated matches the distance between the edge of the stent graft with greater curvature and the opening (which corresponds to the "shortest distance Lf" described later). That is to say, the position of the opening is determined so as to satisfy the relation Lf = L1. In a case in which there is no factor that has an effect on the implantation of the stent graft, such an arrangement is capable of providing alignment between the opening and the orifice of the branched vessel in a relatively simple manner.

In contrast, in a case in which there is relatively severe twisting in the blood vessel, because of the cause described above, with such an arrangement in which the position of the opening is determined based on only a single condition of the distance between the edge of the stent graft with greater curvature and the opening matching the distance between the edge of the blood vessel with greater curvature and the orifice of the branched vessel, it is difficult to provide alignment between the opening and the orifice of the branched vessel.

The present inventors have found that, by simulating the insertion of the sheath into a blood vessel based on information with respect to the degree of twisting of the blood vessel, and by determining the position of the opening based on the simulation result, such an arrangement is capable of determining, in a simple manner, an optimal position of the opening to be formed in the tubular wall of the stent graft.

Next, detailed description will be made below regarding an embodiment of the present invention with reference to the drawings. It should be noted that an existing component or the like may be substituted as appropriate for each component employed in the present embodiment. Also, various modifications may be made, examples of which include a combination with other existing components. Accordingly, description of the present embodiment is by no means intended to restrict the technical scope of the present invention described in the appended claims.

First embodiment

Fig. 1 is a block diagram showing a tubular endoprosthesis manufacturing system 100 according to a first embodiment of the present invention. The tubular endoprosthesis manufacturing system 100 is configured to design and manufacture a thoracic aorta stent graft 6 which is used for medical treatment to be applied to a thoracic aortic aneurysm. The tubular endoprosthesis manufacturing system 100 includes a tubular endoprosthesis designing apparatus 1 and a tubular endoprosthesis manufacturing apparatus 2.

The tubular endoprosthesis designing apparatus 1 supports the design of a stent graft 6. The tubular endoprosthesis manufacturing apparatus 2 supports the manufacture of the stent graft 6 based on the design made by the tubular endoprosthesis designing apparatus 1.

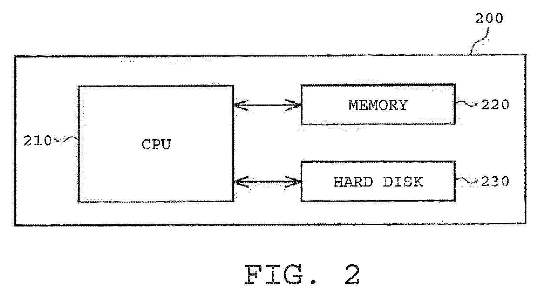

The tubular endoprosthesis designing apparatus 1 includes a three-dimensional model generating unit 10, a simulation unit 20, and an opening position determination unit 30. The three-dimensional model generating unit 10, the simulation unit 20, and the opening position determination unit 30 are each configured as a software component provided by a computer 200 shown in Fig. 2.

Fig. 2 is a block diagram showing the computer 200. The computer 200 is connected to a display, a mouse, and a keyboard. The computer 200 includes a CPU 210, memory (RAM) 220, and a hard disk 230.

The hard disk 230 stores an operating system, a program (tubular endoprosthesis design program) to be used to execute a tubular endoprosthesis designing method for designing the stent graft 6, a correction value table (see Table 1 described later), and the like. It should be noted that the hard disk 230 may preferably be configured as a non-temporary recoding medium. For example, instead of the hard disk 230, non-transitory storage medium such as EPROM, flash memory, or the like, or otherwise a CD-ROM may be employed.

The CPU 210 uses the memory 220 as appropriate so as to read out the data stored in the hard disk 230 and to execute a program or calculation.

Returning to Fig. 1, description will be made. The three-dimensional model generating unit 10 generates, on the computer, a three-dimensional model AA of a blood vessel in which the stent graft 6 is to be implanted, based on the two-dimensional blood vessel images and the three-dimensional blood vessel images of the blood vessel in which the stent graft 6 is to be implanted (which corresponds to a portion in the vicinity of the thoracic aorta of the patient, described in the present embodiment).

Fig. 3 is a diagram showing the three-dimensional model AA. The three-dimensional model AA includes a three-dimensional model of an aneurysm AX that has occurred in the thoracic aorta A1 of the patient, and a three-dimensional model of blood vessels in the vicinity of the aneurysm AX. Three branched vessels, i.e., the brachiocephalic artery A10, the left common carotid artery A20, and the left subclavian artery A30, branch from the thoracic aorta A1. Furthermore, the right common carotid artery A11 and the right subclavian artery A12 branch from the brachiocephalic artery A10.

Fig. 4 is an external perspective view of the stent graft 6. The stent graft 6 is formed by coating a framework, which is called a stent, with a synthetic graft, which is called a graft. The stent has a so-called Z-type stent framework. Specifically, the stent is configured as an approximately cylindrical structure formed by folding back a metal wire in a zigzag manner. The metal wire is formed as a stainless steel wire, a Ni-Ti alloy wire, a titanium alloy wire, or the like, for example. On the other hand, the graft is fixed such that it coats the stent over the external face. The graft is formed of fluorine resin such as PTFE (polytetrafluoroethylene), or polyester resin such as polyethylene terephthalate, for example.

The stent graft 6 is formed such that it is curved in an arched shape, i.e., is curved in a predetermined direction. Furthermore, openings 61, 62, and 63 each having a predetermined shape are formed in the tubular wall of the stent graft 6. The openings 61 through 63 are formed such that they respectively face the orifices of the brachiocephalic artery A10, the left common carotid artery A20, and the left subclavian artery A30, each of which branch from the thoracic aorta A1.

Fig. 5 is a diagram showing a state in which the sheath 5 is inserted into the three-dimensional model AA. In Fig. 5, the positive side in the X direction represents the right side, the positive side in the Y direction represents the lower side, and the positive side in the Z direction represents the back side in the depth direction in the drawing.

The sheath 5 is formed such that it is curved in an approximately J shape, i.e., such that it is curved in a predetermined direction. The sheath 5 is configured as a tubular member formed of a flexible material. The stent graft 6 is housed in the sheath 5 in a state in which the direction in which the stent graft 6 is curved matches the direction in which the sheath 5 is curved.

Returning to Fig. 1, the simulation unit 20 simulates a state in which the sheath 5 is inserted into the three-dimensional model AA generated by the three-dimensional model generating unit 10.

The opening position determination unit 30 determines the position of each of the openings 61 through 63 to be formed in the tubular wall of the stent graft 6, based on the simulation result obtained by the simulation unit 20 and the information with respect to the shape of twisting of the thoracic aorta A1 in a region in which the stent graft 6 is to be implanted. Description will be made below with reference to Figs. 6 through 13 regarding a case in which the opening position determination unit 30 determines the position of the opening 61, which is one from among the aforementioned openings 61 through 63. It should be noted that the opening position determination unit 30 also supports the determination of the position of each of the openings 62 and 63 by means of the same operation as that for determining the position of the opening 61.

Figs. 6 and 7 are diagrams each showing the position relation between an edge line R1 of the thoracic aorta A1 and an edge line R2 of the sheath 5 in a state in which it is inserted into the thoracic aorta A1, which is obtained as a simulation result by the simulation unit 20. In Fig. 6, the positive side in the X direction represents the front side in the depth direction that is orthogonal to the drawing, the positive side in the Y direction represents the lower side, and the positive side in the Z direction represents the right side, which correspond to the directions shown in Fig. 5. In Fig. 7, the positive side in the X direction represents the lower-left side, the positive side in the Y direction represents the back side in the depth direction that is orthogonal to the drawing, and the positive side in the Z direction represents the lower-right side, which correspond to the directions shown in Figs. 5 and 6.

Description will be made below regarding the "twisting of the blood vessel" with reference to the thoracic aorta A1 as an example shown in Fig. 5. A "state in which the blood vessel has no twisting" represents a state in which the thoracic aorta A1, which curves in an inverted U shape, has a center axis extending on the XY plane. That is to say, a "state in which the blood vessel has twisting" represents a state in which the curved thoracic aorta A1 has a center axis that deviates from the XY direction, i.e., a state in which the thoracic aorta A1 has a curvature that curves in a direction having a vector component that is orthogonal to the XY plane (i.e., in a direction along the Z axis).

The "edge line R1 of the thoracic aorta A1" represents the line that passes through the side of the thoracic aorta A1 with the greatest curvature. The "edge line R2 of the sheath 5" represents the line that passes through the side of the sheath 5 with the greatest curvature. The "edge line R3 of the stent graft 6" represents the line that passes through the side of the stent graft 6 with the greatest curvature. Here, the "line that passes through the side with the greatest curvature" represents a line that passes through the outermost point of each slice portion that forms a curved object.

First, as shown in Fig. 7, the opening position determination unit 30 instructs the simulation unit 20 to calculate, as a simulation result, the shortest distance L1 between the edge line R1 of the thoracic aorta A1 in a region in which the stent graft 6 is to be implanted and an edge point P1 of the orifice of the brachiocephalic artery A10. The "edge point P1" represents the position used as a reference point on the edge of the orifice of the brachiocephalic artery A10. Also, a "tangent line RP1" represents a tangent line to the edge of the orifice of the brachiocephalic artery A10 that passes through the edge point P1, which is in parallel with the edge line R1 of the thoracic aorta A1.

Next, the opening position determination unit 30 determines a position deviation L2 between the edge line R1 of the thoracic aorta A1 in a region in which the stent graft 6 is to be implanted and the edge line R2 of the sheath 5 in a state in which the sheath 5 is inserted into this region. Specifically, the opening position determination unit 30 calculates, as the position deviation L2, the shortest distance between the edge line R1 of the thoracic aorta A1 and the edge line R2 of the sheath 5 in the vicinity of the aforementioned edge point P1.

Next, the opening position determination unit 30 acquires a correction value L4 that corresponds to the position deviation L2 with reference to a correction value table shown in the following Table 1.

Table 1

| | L2 [mm] | L4 [mm] |
|---|---|---|
| IN A CASE IN WHICH EDGE LINE R2 OF SHEATH 5 DEVIATES FROM EDGE LINE R1 OF THORACIC AORTAA1 IN POSITIVE DIRECTION ALONG Z AXIS | 12≦L2 | 12.5 |
| | 6≦L2< 12 | 7.5 |
| | 0<L2<6 | 2.5 |
| IN A CASE IN WHICH EDGE LINE R2 OF SHEATH 5 MATCHES EDGE LINE R1 OF THORACIC AORTAA1 IN Z DIRECTION | 0 | 0 |
| IN A CASE IN WHICH EDGE LINE R2 OF SHEATH 5 DEVIATES FROM EDGE LINE R1 OF THORACIC AORTAA1 IN NEGATIVE DIRECTION ALONG Z AXIS | 0<L2<6 | -2.5 |
| | 6≦L2<12 | -7.5 |
| | 12≦L2 | -12.5 |

It should be noted that the aforementioned position deviation L2 becomes larger according to an increase in the degree of twisting of the thoracic aorta A1. That is to say, the position deviation L2 indirectly represents the degree of twisting of the thoracic aorta A1. Accordingly, the position deviation L2 can be used by the opening position determination unit 30 as the information with respect to the shape of twisting of the thoracic aorta A1 in a region in which the stent graft 6 is to be implanted.

Next, the opening position determination unit 30 substitutes the aforementioned shortest distance L1 and the aforementioned correction value L4 into the following Expression (3), so as to calculate a shortest distance Lf between the edge line R3 of the stent graft 6 and an edge point Q1 of the opening 61. The edge point Q1 represents a reference point defined on the edge of the opening 61. Also, a tangent line RQ1 represents a tangent line to the edge of the opening 61 defined such that it passes through the edge point Q1, and such that is in parallel with the edge line R3 of the stent graft 6 (see Figs. 9, 11, and 13, described later).

Expression 3

$$Lf = L1 + L4 \quad \cdots \quad (3)$$

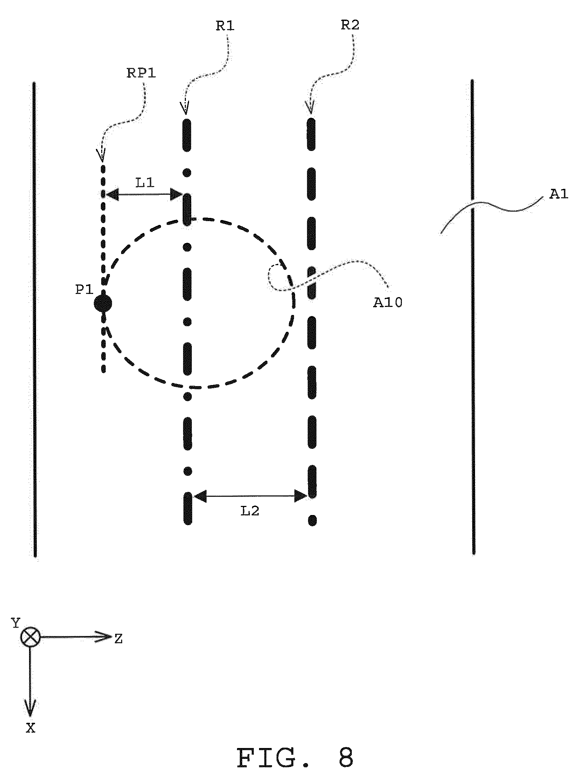

Description will be made below with reference to Figs. 8 through 13 regarding the relation between: the position relation between the edge line R1 of the thoracic aorta A1 and the edge line R2 of the sheath 5 calculated as the simulation result by the simulation unit 20; and the position relation between the edge line R3 of the stent graft 6 and the edge point Q1 of the opening 61.

Fig. 8 is a diagram showing the position relation between the edge line R1 of the thoracic aorta A1 and the edge line R2 of the sheath 5 calculated as the simulation result by the simulation unit 20 in a case in which the edge line R2 of the sheath 5 deviates from the edge line R1 of the thoracic aorta A1 in the positive direction along the Z axis. Fig. 9 is a diagram showing the position relation between the edge line R3 of the stent graft 6 and the edge point Q1 of the opening 61 in a case as shown in Fig. 8.

As shown in Fig. 8, in a case in which the edge line R2 of the sheath 5 deviates from the edge line R1 of the thoracic aorta A1 in the positive direction along the Z axis, the edge point Q1 of the opening 61 deviates from the edge line R3 of the stent graft 6 in the negative direction along the Z axis as shown in Fig. 9.

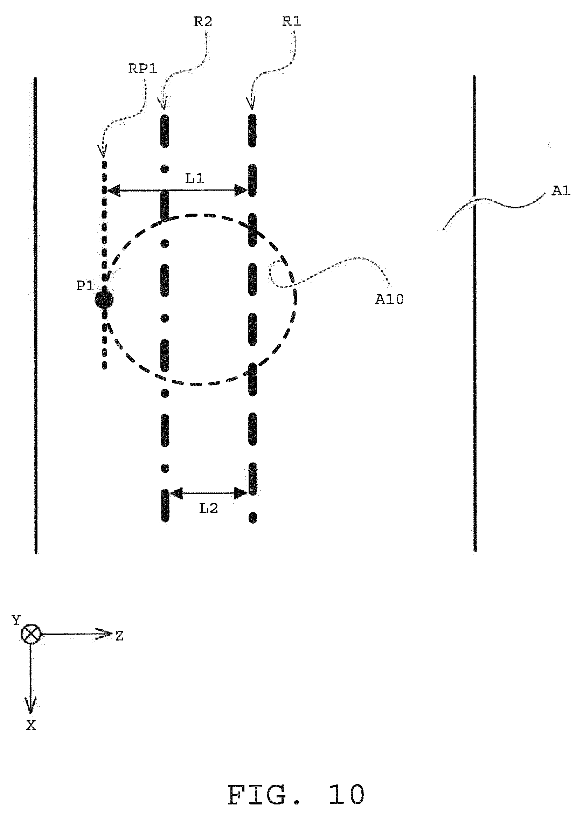

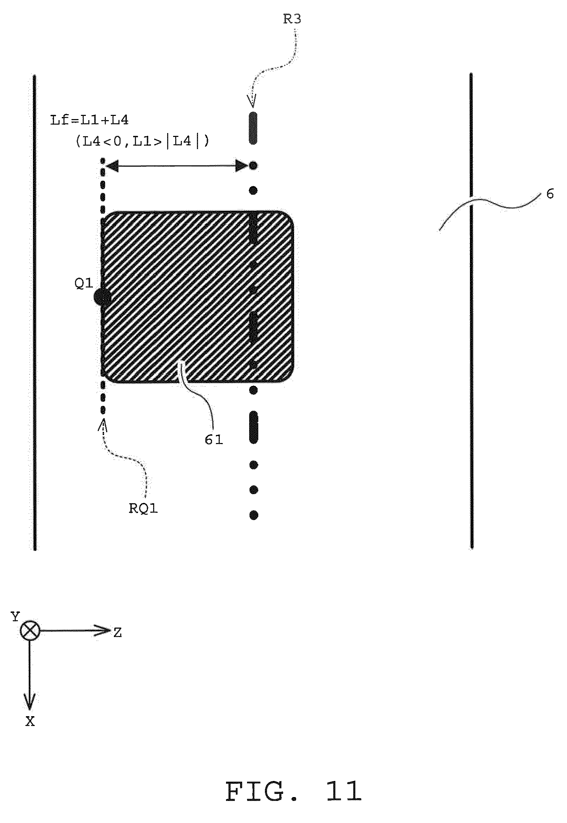

Fig. 10 is a diagram showing the position relation between the edge line R1 of the thoracic aorta A1 and the edge line R2 of the sheath 5 calculated as the simulation result by the simulation unit 20 in a case in which the edge line R2 of the sheath 5 deviates from the edge line R1 of the thoracic aorta A1 in the negative direction along the Z axis and in a case in which the shortest distance L1 is greater than the absolute value of the correction value L4 (L1 > |L4|). Fig. 11 is a diagram showing the position relation between the edge line R3 of the stent graft 6 and the edge point Q1 of the opening 61 in a case as shown in Fig. 10.

In a case in which the edge line R2 of the sheath 5 deviates from the edge line R1 of the thoracic aorta A1 in the negative direction along the Z axis and in a case in which the shortest distance L1 is greater than the absolute value of the correction value L4 as shown in Fig. 10, the edge point Q1 of the opening 61 deviates from the edge line R3 of the stent graft 6 in the negative direction along the Z axis as shown in Fig. 11.

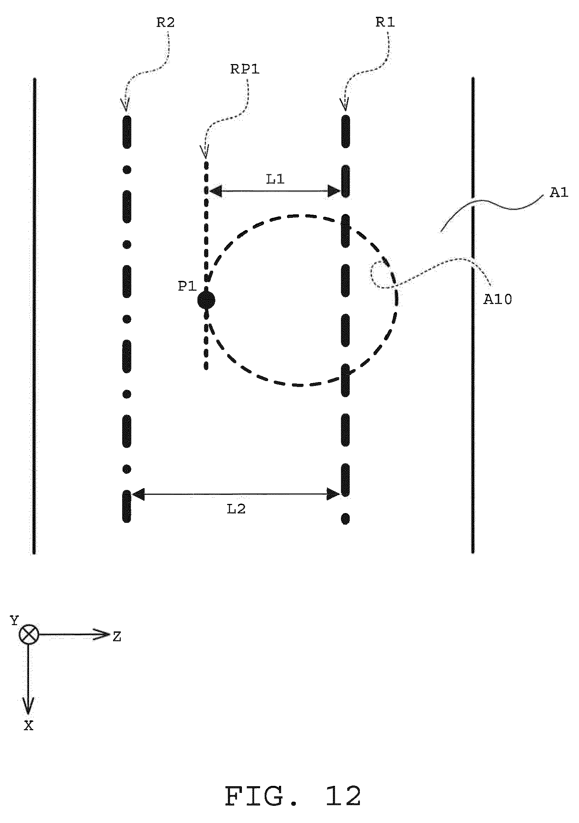

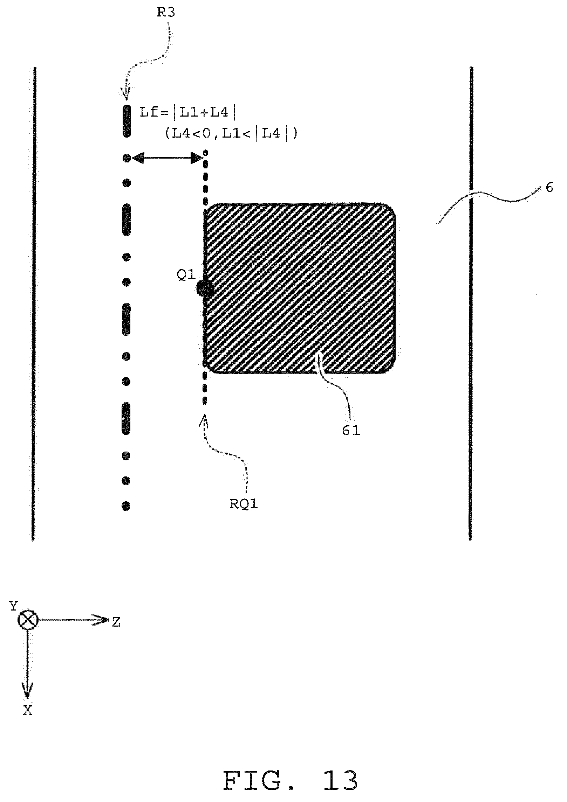

Fig. 12 is a diagram showing the position relation between the edge line R1 of the thoracic aorta A1 and the edge line R2 of the sheath 5 calculated as the simulation result by the simulation unit 20 in a case in which the edge line R2 of the sheath 5 deviates from the edge line R1 of the thoracic aorta A1 in the negative direction along the Z axis and in a case in which the shortest distance L1 is smaller than the absolute value of the correction value L4 (L1 < |L4|). Fig. 13 is a diagram showing the position relation between the edge line R3 of the stent graft 6 and the edge point Q1 of the opening 61 in a case as shown in Fig. 12.

In a case in which the edge line R2 of the sheath 5 deviates from the edge line R1 of the thoracic aorta A1 in the negative direction along the Z axis and in a case in which the shortest distance L1 is smaller than the absolute value of the correction value L4 as shown in Fig. 12, the edge point Q1 of the opening 61 deviates from the edge line R3 of the stent graft 6 in the positive direction along the Z axis as shown in Fig. 13.

Fig. 14 is a flowchart showing a manufacturing method for the stent graft 6 (manufacturing method for a tubular endoprosthesis according to the first embodiment) provided by the tubular endoprosthesis manufacturing system 100.

The tubular endoprosthesis manufacturing apparatus 2 performs membrane member preparation step (Step S1), following which the flow proceeds to Step S2. In the membrane member preparation step, the tubular endoprosthesis manufacturing apparatus 2 prepares a membrane member to be used as a graft. It should be noted that the membrane member thus prepared is configured as a membrane sheet, and is cut in a predetermined size according to a standard specification, for example.

The tubular endoprosthesis designing apparatus 1 performs opening position determination step (Step S2), following which the flow proceeds to Step S3. In the opening position determination step, the tubular endoprosthesis designing apparatus 1 determines the position of each of the openings 61 through 63 to be formed in the membrane member prepared in Step S1, details of which will be described later with reference to Fig. 15.

The tubular endoprosthesis manufacturing apparatus 2 performs opening formation step (Step S3), following which the flow proceeds to Step S4. In the opening formation step, the tubular endoprosthesis manufacturing apparatus 2 forms each of the openings 61 through 63 in the membrane member prepared as a sheet member in Step S1 at a position determined in Step S2 such that they conform to the standard specification. Examples of a method for forming each of the openings 61 through 63 include punching using a press die.

The tubular endoprosthesis manufacturing apparatus 2 performs graft formation step (Step S4), following which the flow proceeds to Step S5. In the graft formation step, the tubular endoprosthesis manufacturing apparatus 2 joins both ends of the membrane sheet member in which the openings 61 through 63 have been formed in Step S3, so as to form a cylindrical graft. Examples of such a method for joining both ends of the membrane sheet member include bonding using an adhesive agent, pressure bonding, welding, and suturing using thread.

The tubular endoprosthesis manufacturing apparatus 2 performs graft fixing step (Step S5), thereby completing the manufacturing of the stent graft 6 shown in Fig. 14. In the graft fixing step, the tubular endoprosthesis manufacturing apparatus 2 arranges a stent within the cylindrical graft formed in Step S4 in a state in which the size of the stent is reduced using a jig, so as to fix the graft around the stent. Examples of a method for fixing the graft around the stent include suturing using thread, bonding, and the like.

Fig. 15 is a flowchart showing the aforementioned opening formation step provided by the tubular endoprosthesis designing apparatus 1.

The tubular endoprosthesis designing apparatus 1 instructs the three-dimensional model generating unit 10 to perform a first step (Step S11), following which the flow proceeds to Step S12. In the first step, the tubular endoprosthesis designing apparatus 1 instructs the three-dimensional model generating unit 10 to generate a three-dimensional model AA of a blood vessel in which the stent graft 6 is to be implanted.

The tubular endoprosthesis designing apparatus 1 instructs the simulation unit 20 to perform a second step (Step S12), following which the flow proceeds to Step S13. In the second step, the tubular endoprosthesis designing apparatus 1 instructs the simulation unit 20 to simulate a state in which the sheath 5 is inserted into the three-dimensional model AA generated in Step S11.

The tubular endoprosthesis designing apparatus 1 instructs the opening position determination unit 30 to perform a third step (Step S13), following which the flow returns to Step S3 shown in Fig. 14. In the third step, the tubular endoprosthesis designing apparatus 1 instructs the opening position determination unit 30 to determine each of the positions of the openings 61 through 63 to be formed in the tubular wall of the stent graft 6, based on the simulation result obtained in Step S3 and the information with respect to the shape of twisting of the thoracic aorta A1 in a region in which the stent graft 6 is to be implanted.

The tubular endoprosthesis designing apparatus 1 as described provides the following advantages.

The tubular endoprosthesis designing apparatus 1 instructs the simulation unit 20 to simulate a state in which the sheath 5 is inserted into the three-dimensional model AA generated by the three-dimensional model generating unit 10. Furthermore, the tubular endoprosthesis designing apparatus 1 instructs the opening position determination unit 30 to determine each of the positions of the openings 61 through 63 to be formed in the tubular wall of the stent graft 6, based on the simulation result obtained by the simulation unit 20 and the information with respect to the shape of twisting of the thoracic aorta A1 in a region in which the stent graft 6 is to be implanted. Thus, even if there is an effect on the implantation position (deviation) of the stent graft 6 due to individual differences in the shape of twisting of the thoracic aorta A1 among patients, such an arrangement is capable of determining an optimal position for each of the openings 61 through 63 giving consideration to this effect due to such individual differences. Such an arrangement is capable of determining, in a simple manner, the optimal position for each of the openings 61 through 63 to be formed in the stent graft 6.

Furthermore, in the tubular endoprosthesis designing apparatus 1, the opening position determination unit 30 uses the information with respect to the shape of twisting of the thoracic aorta A1 (information with respect to the shape of curvature of the thoracic aorta A1 in a region in which the stent graft 6 is to be implanted). Thus, the tubular endoprosthesis designing apparatus 1 is capable of determining an optimal position for each of the openings 61 through 63 to be formed in the tubular wall of the stent graft 6 giving consideration to the shape of twisting of the thoracic aorta A1.

Moreover, the tubular endoprosthesis designing apparatus 1 is configured to determine each of the positions of the openings 61 through 63 based on the position relation between the edge line R1 of the thoracic aorta A1 in a region in which the stent graft 6 is to be implanted and the edge line R2 of the sheath 5 in a state in which it is inserted into this region. Thus, such an arrangement is capable of determining an optimal position for each opening regardless of the degree of twisting (degree of curvature) of the blood vessel.

Moreover, the tubular endoprosthesis designing apparatus 1 is capable of determining an optimal position for each of openings 61 through 63 in a relatively simple manner using the aforementioned Table 1 and Expression (3).

With the tubular endoprosthesis manufacturing system 100 including the tubular endoprosthesis designing apparatus 1 and the tubular endoprosthesis manufacturing apparatus 2 as described above, such an arrangement is capable of manufacturing the stent graft 6 having the openings 61 through 63 each formed at an optimal position.

Second embodiment

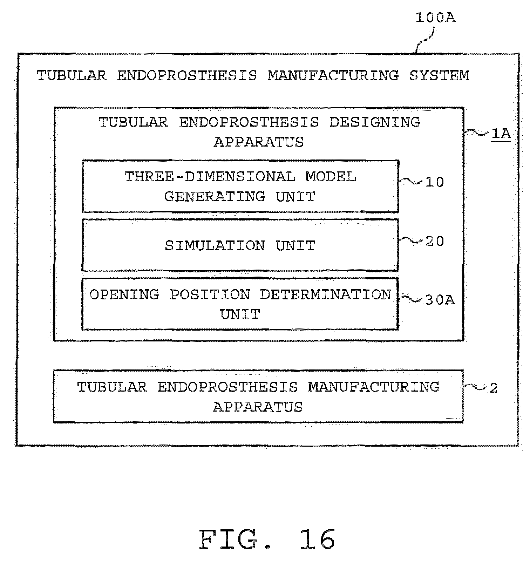

Fig. 16 is a block diagram showing a tubular endoprosthesis manufacturing system 100A according to a second embodiment of the present invention. The tubular endoprosthesis manufacturing system 100A has the same configuration as that of the tubular endoprosthesis manufacturing system 100 according to the first embodiment of the present invention described with reference to Fig. 1, except that the tubular endoprosthesis manufacturing system 100A includes a tubular endoprosthesis designing apparatus 1A instead of the tubular endoprosthesis designing apparatus 1. The tubular endoprosthesis designing apparatus 1A has the same configuration as that of the tubular endoprosthesis designing apparatus 1, except that the tubular endoprosthesis designing apparatus 1A includes an opening position determination unit 30A instead of the opening position determination unit 30. It should be noted that, in the following description of the tubular endoprosthesis manufacturing system 100A, the same components as those of the tubular endoprosthesis manufacturing system 100 are denoted by the same reference symbols, and description thereof will be omitted.

The opening position determination unit 30A determines each of the positions of the openings 61 through 63 to be formed in the tubular wall of the stent graft 6, based on the simulation result obtained by the simulation unit 20 and the information with respect to the shape of twisting of the thoracic aorta A1 in a region in which the stent graft 6 is to be implanted, in the same manner as the opening position determination unit 30. It should be noted that the opening position determination unit 30A uses the Expression (4) described below instead of the aforementioned Table 1 and Expression (3).

Specifically, first, the opening position determination unit 30A instructs the simulation unit 20 to calculate, as a simulation result, the shortest distance L1 between the edge line R1 of the thoracic aorta A1 in a region in which the stent graft 6 is to be implanted and the edge point P1 of the orifice of the brachiocephalic artery A10, in the same manner as the opening position determination unit 30.

Next, the opening position determination unit 30A instructs the simulation unit 20 to calculate, as a simulation result, the position deviation L2 between the edge line R1 of the thoracic aorta A1 in a region in which the stent graft 6 is to be implanted and the edge line R2 of the sheath 5 in a state in which it is inserted into this region, in the same manner as the opening position determination unit 30.

In a case in which there is relatively severe twisting in the thoracic aorta A1, in some cases, such an arrangement leads to misalignment, i.e., deviation between the edge line R3 of the stent graft 6 and the edge line R2 of the sheath 5 after the stent graft 6 is deployed from the sheath 5.

In order to solve such a problem, as a next step, the opening position determination unit 30A instructs the simulation unit 20 to calculate, as a simulation result, a position deviation L3 between the edge line R2 of the sheath 5 in a state in which it is inserted into a region in which the stent graft 6 is to be implanted and the edge line R3 of the stent graft 6 in a state in which it is implanted after it is deployed to this region from the sheath 5.

As a method for calculating the position deviation L3, for example, a state of the stent graft 6 may be simulated from the time point at which it is deployed from the sheath 5 up to the time point at which the stent graft 6 is implanted. In the simulation, the position deviation may be calculated between the edge line R2 of the sheath 5 and the edge line R3 of the stent graft 6 in a state in which the stent graft 6 is implanted (distance between the edge line R2 of the sheath 5 and the edge line R3 of the stent graft 6 along an arc defined on a virtual cross-sectional plane that includes a section of the sheath 5 and a section of the stent graft 6 and that is orthogonal to the center axis of the thoracic aorta A1). As an another method for obtaining the position deviation L3, a relation expression between the position deviation L3 and the position deviation L2 may be derived based on measurement data, and the position deviation L3 may be obtained using the relation expression thus derived.

The opening position determination unit 30A substitutes the aforementioned shortest distance L1 and the aforementioned position deviations L2 and L3 into the following Expression (4), so as to calculate the shortest distance Lf between the edge line R3 of the stent graft 6 and the edge point Q1 of the opening 61.

Expression 4

$$Lf = L1 + L2 + L3 \quad \cdots \quad (4)$$

With the tubular endoprosthesis designing apparatus 1A described above using Expression (4) instead of the aforementioned Table 1 and Expression (3), such an arrangement provides the same effects as those provided by the tubular endoprosthesis designing apparatus 1.

As the information with respect to the shape of twisting of the thoracic aorta A1 in a region in which the stent graft 6 is to be implanted, the tubular endoprosthesis designing apparatus 1A instructs the opening position determination unit 30A to use the following two position relations calculated as the simulation results by the simulation unit 20. One of the aforementioned two position relations is the position relation between the edge line R1 of the thoracic aorta A1 in a region in which the stent graft 6 is to be implanted and the edge line R2 of the sheath 5 in a state in which it is inserted into this region. The other of the aforementioned two position relations is the position relation between the edge line R2 of the sheath 5 in a state in which it is inserted into a region in which the stent graft 6 is to be implanted and the edge line R3 of the stent graft 6 in a state in which it is implanted in this region after it is deployed from the sheath 5. With such an arrangement, each of the positions of the openings 61 through 63 is determined using the aforementioned two position relations. Thus, such an arrangement is capable of determining a further optimal position for each of the openings 61 through 63 regardless of the degree of twisting (degree of curvature) of the thoracic aorta A1.

Detailed description has been made with reference to the drawings regarding the embodiments of the present invention. However, such a specific configuration is not restricted to the aforementioned embodiments. Also, various kinds of design changes may be made, which are encompassed within the technical scope of the present invention.

For example, description has been made above in the aforementioned embodiments with reference to the stent graft as an example of a tubular endoprosthesis. However, the application of the present invention is not restricted to such an arrangement. Also, the present invention is applicable to other kinds of tubular endoprostheses to be implanted in the body, examples of which include a synthetic graft and the like. It should be noted that examples of such a stent graft include a stent graft for the abdominal aorta and a stent graft for the thoracoabdominal aorta, in addition to the stent graft for the thoracic aorta. Also, such a stent graft is not restricted to an arrangement in which a stent is arranged within a graft (a so-called endoskeletal structure). Also, such a stent graft may be configured as an arrangement in which a stent is arranged on the outer side of a graft (a so-called exoskeletal structure). Also, such a stent graft may be configured as an arrangement in which a graft is adhered to each of the inner face and the outer face of the stent.

Description has been made in the aforementioned embodiments regarding an aneurysm as an example of a lesion. However, the lesion to which the present invention is applicable is not restricted to such an aneurysm. For example, the present invention is applicable to other kinds of lesions such as varicose veins, narrowed blood vessels, etc. It should be noted that examples of the aforementioned aneurysm include an aortic aneurysm, cerebral aneurysm, peripheral aneurysm (popliteal aneurysm, iliac aneurysm, etc.). Examples of the aforementioned varicose veins include a varix of the lower extremity.

Description has been made in the aforementioned embodiments regarding the stent graft 6 configured such that it is curved in a predetermined direction. However, the present invention is not restricted to such an arrangement. Also, the stent graft 6 may be configured to have a straight structure (linear structure) as a basic structure and to have a portion that can be curved in a predetermined direction. Also, the stent graft 6 may be configured to have a portion that can be curved in various directions.

Description has been made in the aforementioned embodiments regarding the sheath 5 configured such that it is curved in a predetermined direction. However, the present invention is not restricted to such an arrangement. Also, the sheath 5 may be configured to have a straight structure (linear structure) as a basic structure and to have a portion that can be curved in a predetermined direction.

Description has been made in the aforementioned embodiments regarding an arrangement in which the three-dimensional model AA is generated by the three-dimensional model generating unit 10 provided as a software component by means of the computer 200. That is to say, the three-dimensional model AA is configured as a virtual model on the computer. However, the present invention is not restricted to such an arrangement. For example, the three-dimensional model AA may be configured as a model formed of a material by means of a 3D printer.

Description has been made in the aforementioned embodiments regarding an arrangement in which, after the position is determined for each of the openings 61 through 63, the openings 61 through 63 are each formed in the membrane sheet member at the corresponding position thus determined. However, the present invention is not restricted to such an arrangement. Also, the openings 61 through 63 may each be formed in a cylindrical graft at a predetermined position. In this case, position alignment may be made between the cylindrical graft and the stent based on the shortest distance Lf calculated using the aforementioned Expression (3) or otherwise Expression (4), so as to fix the graft to the stent.

Description has been made in the aforementioned embodiments regarding an arrangement in which, in the determination of the position of the opening 61, the edge point P1, which is defined as a single reference point on the orifice of the brachiocephalic artery A10, is used as the reference position of the orifice of the brachiocephalic artery A10. However, the present invention is not restricted to such an arrangement. Also, multiple edge points defined on the orifice of the brachiocephalic artery A10 may be used as the reference positions. In this case, the same number of edge points may be defined on the edge of the opening 61, and the multiple edge points thus defined may be employed as the reference positions of the opening 61, instead of employing the single edge point Q1 defined on the edge of the opening 61.

Description has been made in the aforementioned embodiments regarding an arrangement in which, in determination of the position of the opening 61, the edge point P1 defined on the edge of the orifice of the brachiocephalic artery A10 is employed as a reference position of the orifice of the brachiocephalic artery A10. However, the present invention is not restricted to such an arrangement. Also, a central position of the orifice of the brachiocephalic artery A10 may be employed as the reference position. In this case, a central portion of the opening 61 may be employed as its reference position instead of employing the edge point Q1 defined on the edge of the opening 61.

Description has been made in the aforementioned embodiments regarding an arrangement in which a single reference position is defined for each of the three branched vessels, and a single reference position is defined for each of the openings 61 through 63. Subsequently, the opening position is determined for each of the openings 61 through 63. However, the present invention is not restricted to such an arrangement. For example, a single reference position may be defined for all three branched vessels in the same manner as in a coordinate system. In this case, after a single reference position is determined for all the openings 61 through 63, the opening positions of the openings 61 through 63 may be determined at the same time.

Also, in the aforementioned first embodiment, the correction value table may be updated as appropriate.

Description has been made in the aforementioned embodiments regarding an arrangement in which the tubular endoprosthesis manufacturing apparatus 2 performs the membrane member preparation step, the opening formation step, the graft formation step, and the graft fixing step, as an example. However, such steps may be performed manually without employing the tubular endoprosthesis manufacturing apparatus. Description has been made in the aforementioned embodiments regarding an arrangement in which the tubular endoprosthesis designing apparatus 1 or 1A performs the opening position determination step (first step through third step). Also, an arrangement may be made without involving such a tubular endoprosthesis designing apparatus. For example, in the first step, such a three-dimensional model of the blood vessels may be formed as a transparent or translucent model (mock-up). Also, the following second and third steps may be performed using the three-dimensional model thus formed.

Reference Numerals

100, 100A tubular endoprosthesis manufacturing system, 1, 1A tubular endoprosthesis designing apparatus, 2 tubular endoprosthesis manufacturing apparatus, 5 sheath, 6, 6A stent graft, 61, 62, 63 opening, 10 three-dimensional model generating unit, 20 simulation unit, 30, 30A opening position determination unit, 200 computer, 210 CPU, 220 memory, 230 hard disk, AA three-dimensional model, A1 thoracic aorta, A10 brachiocephalic artery, A11 right common carotid artery, A12 right subclavian artery, A20 left common carotid artery, A30 left subclavian artery, AX aneurysm, P1 edge point of the orifice of the brachiocephalic artery, R1 edge line of the thoracic aorta, R2 edge line of the sheath, R3 edge line of the stent graft, Q1 edge point of the opening.

**Claims**

1. A tubular endoprosthesis designing apparatus (1) that designs a tubular endoprosthesis (6) to be implanted in a body after it is housed in a sheath (5), wherein the tubular endoprosthesis is to be implanted in a region including an orifice of a branched vessel,
   wherein the tubular endoprosthesis designing apparatus comprises:

   a three-dimensional model generating unit (10) that generates a three-dimensional model (AA) of a blood vessel in which the tubular endoprosthesis is to be implanted;
   a simulation unit (20) that simulates a state in which the sheath is inserted into the three-dimensional model generated by the three-dimensional model generating unit; and
   an opening position determination unit (30) that determines a position of an opening (61, 62, or 63) to be formed in a tubular wall of the tubular endoprosthesis, based on a simulation result obtained by the simulation unit and information with respect to a shape of curvature of the blood vessel in which the tubular endoprosthesis is to be implanted,
   wherein the sheath is configured such that it is curved in a predetermined single direction or otherwise can be curved,
   and wherein the information used by the opening position determination unit is a position relation between an edge line (R1) of the blood vessel in a region in which the tubular endoprosthesis is to be implanted and an edge line (R2) of the sheath in a state in which it is inserted into the aforementioned region, which is obtained as a simulation result by the simulation unit.

2. The tubular endoprosthesis designing apparatus according to claim 1, wherein the opening position determination unit defines, as a twisting of a blood vessel, a deviation of the blood vessel in a direction that is orthogonal to a direction of curvature of the blood vessel is curved,
   and wherein the opening position determination unit uses, as a shape of curving of the blood vessel in the region in which the tubular endoprosthesis is to be implanted, a shape of twisting of the blood vessel in the region in which the tubular endoprosthesis is to be implanted.

3. The tubular endoprosthesis designing apparatus according to claim 1 or 2, wherein the tubular endoprosthesis is configured such that it is curved in a predetermined direction or otherwise such that it can be curved,
   wherein the tubular endoprosthesis is housed in the sheath in a state in which the direction in which the tubular endoprosthesis is curved matches the direction in which the sheath is curved,
   and wherein the information used by the opening position determination unit comprises:

   a position relation between an edge line (R1) of a blood vessel in a region in which the tubular endoprosthesis is to be implanted and an edge line (R2) of the sheath in a state in which it is inserted into the aforementioned region, which is obtained as a simulation result by the simulation unit; and
   a position relation between an edge line (R2) of the sheath in a state in which it is inserted into a region in which the tubular endoprosthesis is to be implanted and an edge line (R3) of the tubular endoprosthesis in a state in which it is implanted in the aforementioned region after it is deployed from the sheath, which is obtained as a simulation result by the simulation unit.

4. The tubular endoprosthesis designing apparatus according to claim 3, wherein the opening position determination unit defines, as L1, a shortest distance between an edge line (R1) of a blood vessel in a region in which the tubular endoprosthesis is to be implanted and a reference position defined on an orifice of a branched vessel, which is obtained as a simulation result by the simulation unit,
   wherein the opening position determination unit defines, as L2, a position deviation between an edge line (R1) of the blood vessel in a region in which the tubular endoprosthesis is to be implanted and an edge line (R2) of the sheath in a state in which it is inserted into the aforementioned region, which is obtained as a simulation result by the simulation unit,
   wherein the opening position determination unit defines, as L3, a position deviation between an edge line (R2) of the sheath in a state in which it is inserted into a region in which the tubular endoprosthesis is to be implanted and an edge line (R3) of the tubular endoprosthesis in a state in which it is implanted in the aforementioned region after it is deployed from the sheath, which is obtained as a simulation result by the simulation unit,
   and wherein the opening position determination unit determines a position of the opening such that a shortest distance Lf between an edge line of the tubular endoprosthesis in a state in which it is implanted in the aforementioned region and a reference position defined on the opening satisfy the following expression (Expression 1):

$$Lf = L1 + L2 + L3 \quad \cdots \quad (1)$$

5. The tubular endoprosthesis designing apparatus according to claim 1 or 2, the opening position determination unit defines, as L1, a shortest distance between an edge line (R1) of a blood vessel in a region in which the tubular endoprosthesis is to be implanted and a reference position defined on an orifice of a branched vessel, which is obtained as a simulation result by the simulation unit,
wherein the opening position determination unit defines, as L2, a position deviation between an edge line (R1) of the blood vessel in a region in which the tubular endoprosthesis is to be implanted and an edge line (R2) of the sheath in a state in which it is inserted into the aforementioned region, which is obtained as a simulation result by the simulation unit,
wherein the opening position determination unit determines a correction value L4 based on the position deviation L2, and wherein the opening position determination unit determines a position of the opening such that a shortest distance Lf between an edge line of the tubular endoprosthesis in a state in which it is implanted in the aforementioned region and a reference position defined on the opening satisfy the following expression (Expression 2):

$$Lf = L1 + L4 \quad \cdots \quad (2)$$

6. A manufacturing method for a tubular endoprosthesis (6) to be implanted in a body after being housed in a sheath (5), wherein the sheath is configured such that it is curved in a predetermined single direction or otherwise such that it can be curved,
wherein the tubular endoprosthesis is to be implanted in a region including an orifice of a branched vessel,
wherein the manufacturing method comprises:

a membrane member preparation step (S1) in which a membrane member to be configured as the tubular endoprosthesis is prepared;
an opening position determination step (S2) in which a position is determined for an opening (61, 62, or 63) to be formed in a tubular wall of the membrane member;
an opening formation step (S3) in which the opening is formed in the membrane member prepared in the membrane member preparation step such that it is formed at a position determined in the opening position determination step,

wherein the opening position determination step comprises:

a first step (S11) in which a three-dimensional model (AA) is generated for a blood vessel in which the tubular endoprosthesis is to be implanted;
a second step (S12) in which a state is simulated in which the sheath is inserted into the three-dimensional model generated in the first step; and
a third step (S13) in which the position of the opening is determined based on a simulation result obtained in the second step and information with respect to a shape of curvature of the blood vessel in which the tubular endoprosthesis is to be implanted,

and wherein the information used in the third step is a position relation between an edge line (R1) of the blood vessel in a region in which the tubular endoprosthesis is to be implanted and an edge line (R2) of the sheath in a state in which it is inserted into the aforementioned region, which is obtained as a simulation result in the second step.

7. A tubular endoprosthesis designing program configured to instruct a computer to execute a tubular endoprosthesis designing method for designing a tubular endoprosthesis (6) that is to be implanted in a body and that is to be housed in a sheath (5), wherein the sheath is configured such that it is curved in a predetermined single direction or otherwise such that it can be curved,
wherein the tubular endoprosthesis is to be implanted in a region including an orifice of a branched vessel,
wherein the tubular endoprosthesis designing program executed by the computer comprises:

a first step (S11) in which a three-dimensional model (AA) is generated for a blood vessel in which the tubular endoprosthesis is to be implanted;

a second step (S12) in which a state is simulated in which the sheath is inserted into the three-dimensional model generated in the first step; and

a third step (S13) in which the position of the opening to be formed in a tubular wall of the tubular endoprosthesis is determined based on a simulation result obtained in the second step and information with respect to a shape of curvature of the blood vessel in which the tubular endoprosthesis is to be implanted,

and wherein the information used in the third step is a position relation between an edge line (R1) of the blood vessel in a region in which the tubular endoprosthesis is to be implanted and an edge line (R2) of the sheath in a state in which it is inserted into the aforementioned region, which is obtained as a simulation result in the second step.

100

TUBULAR ENDOPROSTHESIS MANUFACTURING SYSTEM

TUBULAR ENDOPROSTHESIS DESIGNING APPARATUS    1

THREE-DIMENSIONAL MODEL GENERATING UNIT    10

SIMULATION UNIT    20

OPENING POSITION DETERMINATION UNIT    30

TUBULAR ENDOPROSTHESIS MANUFACTURING APPARATUS    2

# FIG. 1

200

```
                                                        200
  ┌─────────────────────────────────────────────────┐
  │                                                   │
  │   ┌───────────────┐      ┌──────────────┐        │
  │   │               │ ←──→ │   MEMORY     │ ～220  │
  │   │               │      └──────────────┘        │
  │210│     CPU       │                               │
  │   │               │      ┌──────────────┐        │
  │   │               │ ←──→ │  HARD DISK   │ ～230  │
  │   └───────────────┘      └──────────────┘        │
  │                                                   │
  └─────────────────────────────────────────────────┘
```

# FIG. 2

FIG. 3

FIG. 4

FIG. 5

FIG. 6

FIG. 7

FIG. 8

R3

RQ1

Lf=L1+L4
(L4>0)

Q1

6

61

Y
Z
X

FIG. 9

FIG. 10

$Lf = L1 + L4$
$(L4 < 0, L1 > |L4|)$

FIG. 11

FIG. 12

R3

$Lf = |L1 + L4|$
$(L4 < 0, L1 < |L4|)$

6

Q1

61

RQ1

Y ⊗ → Z

X

FIG. 13

FIG. 14

```
        ╭─────────────────────────╮
        │ START OPENING POSITION  │
        │   DETERMINATION STEP    │
        ╰─────────────────────────╯
                     │
                     ▼
        ┌─────────────────────────┐ ⟋ S11
        │       FIRST STEP        │
        └─────────────────────────┘
                     │
                     ▼
        ┌─────────────────────────┐ ⟋ S12
        │       SECOND STEP       │
        └─────────────────────────┘
                     │
                     ▼
        ┌─────────────────────────┐ ⟋ S13
        │       THIRD STEP        │
        └─────────────────────────┘
                     │
                     ▼
        ╭─────────────────────────╮
        │         RETURN          │
        ╰─────────────────────────╯
```

# FIG. 15

100A

TUBULAR ENDOPROSTHESIS MANUFACTURING SYSTEM

TUBULAR ENDOPROSTHESIS DESIGNING APPARATUS 〜1A

THREE-DIMENSIONAL MODEL GENERATING UNIT 〜10

SIMULATION UNIT 〜20

OPENING POSITION DETERMINATION UNIT 〜30A

TUBULAR ENDOPROSTHESIS MANUFACTURING APPARATUS 〜2

FIG. 16

Europäisches
Patentamt

European
Patent Office

Office européen
des brevets

# EUROPEAN SEARCH REPORT

Application Number

EP 16 15 8797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2014 057743 A (KAWASUMI LAB INC) 3 April 2014 (2014-04-03) | 1-5 | INV. A61F2/07 A61F2/06 A61B5/00 |
| Y | * the whole document * | 6,7 | |
| Y | DAVID PERRIN ET AL: "Deployment of stent grafts in curved aneurysmal arteries: toward a predictive numerical tool", INTERNATIONAL JOURNAL FOR NUMERICAL METHODS IN BIOMEDICAL ENGINEERING, vol. 31, no. 1, 29 January 2015 (2015-01-29), pages n/a-n/a, XP055287004, ISSN: 2040-7939, DOI: 10.1002/cnm.2698 * the whole document * | 6,7 | |
| A | US 2008/058633 A1 (BOYDEN EDWARD S [US] ET AL) 6 March 2008 (2008-03-06) * the whole document * | 1-7 | |
| A | US 2008/201007 A1 (BOYDEN EDWARD S [US] ET AL) 21 August 2008 (2008-08-21) * the whole document * | 1-7 | TECHNICAL FIELDS SEARCHED (IPC) A61F A61B G06T |
| A | US 2013/296998 A1 (LEOTTA DANIEL F [US] ET AL) 7 November 2013 (2013-11-07) * the whole document * | 1-7 | |

The present search report has been drawn up for all claims

| Place of search | Date of completion of the search | Examiner |
|---|---|---|
| The Hague | 11 July 2016 | Geuer, Melanie |

EPO FORM 1503 03.82 (P04C01)

**ANNEX TO THE EUROPEAN SEARCH REPORT
ON EUROPEAN PATENT APPLICATION NO.**

EP 16 15 8797

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2016

| Patent document cited in search report | | Publication date | Patent family member(s) | | Publication date |
|---|---|---|---|---|---|
| JP 2014057743 | A | 03-04-2014 | NONE | | |
| US 2008058633 | A1 | 06-03-2008 | NONE | | |
| US 2008201007 | A1 | 21-08-2008 | NONE | | |
| US 2013296998 | A1 | 07-11-2013 | US 2013296998 A1 | | 07-11-2013 |
| | | | US 2015234957 A1 | | 20-08-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010227486 A
- JP 2013052282 A